# EUROPEAN PATENT SPECIFICATION

(11) **EP 4 138 666 B1**
(45) Date of publication and mention of the grant of the patent: **04.12.2024**
(21) Application number: 21718605.5
(22) Date of filing: 19.04.2021
(51) Int. Cl.: A61B 5/291

(54) **AN ELECTRODE DEVICE FOR AMPLITUDE-INTEGRATED ELECTROENCEPHALOGRAPHY**
ELEKTRODENVORRICHTUNG FÜR AMPLITUDENINTEGRIERTE ELEKTROENZEPHALOGRAPHIE
DISPOSITIF D'ÉLECTRODE POUR ÉLECTROENCÉPHALOGRAPHIE INTÉGRÉE EN AMPLITUDE

(30) Priority: 20.04.2020 EP 20382317
(43) Date of publication of application: 01.03.2023
(73) Proprietor: Universitat Rovira I Virgili, 43003 Tarragona (ES); Institut d'Investigació Sanitària Pere Virgili, 43003 Tarragona (ES)
(72) Inventor: FABREGAT SANJUAN, Albert, 43007 Tarragona (ES); PASCUAL RUBIO, Vicenç, 43005 Tarragona (ES)
(74) Representative: Ungria López, Javier
(86) International application number: PCT/EP2021/060103
(87) International publication number: WO 2021/213985

(56) References cited:
- CN-A- 107 788 976
- US-A- 4 166 457
- US-A- 4 250 878
- US-A- 5 037 380

## Description

### Technical Field

The present invention is directed, in general, to the field of medical electrodes. In particular, the invention relates to an electrode device designed to record superficial brain bioelectric activity during long periods of time from infants in the neonatal intensive care units (NICU's). The electrode is particularly designed to be used in amplitude-integrated electroencephalography (aEEG), however it can also be used to record other long-term neurophysiological recording tests such as continuous electroencephalography monitoring (CEEG), among others.

### Background of the invention

Amplitude-integrated electroencephalography (aEEG) is an easily accessible technique for monitoring the brain function in infants. In its simplest form, aEEG is a processed single-channel electroencephalogram that is filtered, rectified, enlarged with a semilogarithmic scale and time compressed.

There are several specific technical aspects for infant aEEG in the NICUs to be considered, beginning with the montage and electrode placement, the long-term recordings (several days or weeks), the lying position of the infants in the NICUs, the frequent manipulation by healthcare staff, the characteristic morphology of the head and the fragility of the scalp of preterm and term infants.

The known or commercial electrodes do not fulfill the requirements needed for infants and present artifacts in the electrical neuroactivity readings. For infants in a critical situation puts risk relevant clinical decisions.

The current problem is not well tackled as the available surface electrodes are not properly sized for term and preterm infant's application. Also, they are usually attached to a long wire that connects them to the bioelectric signal amplifier. This long wire electrode hinders the manipulation of the newborn by health care staff. In addition, the electrode wire is usually attached to the electrode laterally. In the lying patient, the usual position in the NICU's, this joint causes a lever force that facilitates the electrode decoupling.

Superficial cup electrodes available have a hole through which the electroconductive gel is replaced in order to maintain its high conductive capacity during long-term recordings. During this process, a blunt-tipped syringe is usually used to remove the old electroconductive gel and apply the new one. At this time, it is usually necessary to apply vertical pressure to beat the conductive gel layer that may have dried on the long-term recordings. This pressure can cause an involuntary contact with the scalp surface, which should be avoided in newborns due to their fragility and proximity to the fontanelles. Also, its rigid consistency (gold or silver electrodes) causes that when they are fixed with adherent tape, the skin indemnity can be put at risk. As well us, the electrodes cover the recording site not offering the opportunity to check the skin of the newborn without detach the electrode.

Disposable adhesive electrodes have the disadvantage of not allowing the replacement of the electroconductive gel and therefore they must be replaced periodically. This involves more manipulation and possible changes in the recording conditions (placement, impedance, etc.), in a situation where a constant monitorization without cuts is a key element.

The subdermal needle electrodes which are often used in the aEEG to achieve low impedance recordings have the disadvantage of producing pain. Pain should always be avoided to improve comfort but in the aEEG it also has the disadvantage of not allowing the accurate assessment of a parameter as important as the latency of onset of the sleep-wake cycle. In addition, the subdermal electrodes have the same drawbacks as most surface electrodes available: are connected to a long wire to the amplifier and are easily detached.

There are known some patents and patents applications in this field.

FR2400370 discloses an electrode device intended to be applied to the skin, for electro-medical purposes. The electrode device can be used for electroencephalography, electrocardiography and electromyography. The electrode comprises a plastics body with a ring-shaped surface that is stuck to the skin by a double-sided tape. An electrode is arranged in a cavity that can be filled with a conductive paste or gel through a hole. The hole is arranged on the axis of the electrode body. At least one discharge duct from the cavity is provided for excess paste or gel, preferably in the form of a groove in the electrode body under the adhesive tape. This electrode has the disadvantage of having the long cable making it difficult for the healthcare staff to handle the child. The connection between the plate and the cable is lateral, facilitating the uncoupling of the electrode by lever forces in the lying patient. Moreover, this type of electrode device does not avoid the possible contact with the scalp during the replacement of electroconductive gel.

US 4657023-A provides a self-adhering electrode for application on the skin of a patient in which a portion of the electrically conductive layer forms an electrode that is substantially surrounded by a pressure-sensitive layer and the remainder of the conductive layer is covered by a substrate which is also conductive and sufficiently pliant to permit the electrode to adjust to the body contour. This electrode does not allow electrode conductive gel replacement without removing the electrode. It has a long wire laterally connected that complicates the manipulation of the infant and facilitates the uncoupling of the electrode by lever forces in the lying patient.

US 4029086-A provides an electrode arrangement which provides for a reliable electrical contact with the skin of a person or animal. The electrode arrangement comprises, in combination, a central post with a base flange, an adhesive pad having a central aperture for receiving the central post, a gel member and a resilient o-ring shaped member. An alternate embodiment comprises a central post and base flange, an adhesive pad having a central aperture for receiving the central post, and a shield washer, with a central aperture for receiving the central post, mounted above the adhesive pad. Both embodiments minimize the effect of physical forces placed on the electrical contact. This electrode has the same disadvantages as US 4657023-A.

US 4166457-A discloses a dry-state bioelectrode having a self-sealing receptacle for receiving electrolyte and/or medicament fluid contents. The receptacle is attached at its opening to a sheet-like flexible base member having an opening in common with the receptacle opening, the bottom of the base member being adapted for fixation at a skin surface. A portion of the skin surface is exposed to the fluid contents of the receptacle through the common opening. An injection site communicates through the wall of the receptacle and provides controlled access for filling. Upon completion of filling, the receptacle self-seals, retaining the fluid contents therein for application of iontophoresis treatment or other procedures requiring use of a potential gradient. An electrode plate is supported at an interior surface of the receptacle for supplying the desired electric potential. This bioelectrode does not have a first protruding part arranged inclined at an angle between 50-80º with respect to the contact surface of the bioelectrode (i.e. the surface of the bioelectrode contacting the skin surface of the patient).

For at least these reasons, a new electrode device which solves the problems of current electrodes is needed.

### Description of the Invention

To that end, the present invention proposes, according to one aspect, an electrode device for aEEG according to claim 1.

As known in the field, the electrode device comprises a housing fixed on an adhesive element to adhere the electrode device to the skin surface of a patient, particularly the scalp; an electrode plate placed in the housing at a certain distance from a support surface of the housing forming a hollow space; and a wire connection for the electrode.

The housing has two protruding parts, a first protruding part and a second protruding part. The first protruding part includes or provides a first hole. The second protruding part includes or provides a second hole for the introduction of the wire connection, so that the wire connection can be attached, e.g. by means of one or more welding points, to the electrode plate. The second protruding part can also have the function of allowing the flow of the electroconductive gel from the hollow space between the electrode plate and the skin to the exterior of the housing to drain the gel from first protruding part to the second one. The second protruding part is disposed, substantially, on the axis of the housing. Therefore, the wire connection is maintained perpendicular or almost perpendicular (about 90º) to the skin surface when performing the measures, which allows minimizing the tangential forces that favor the inclination of the electrode plate and it's uncoupling from the scalp. Likewise, the electrode plate is placed horizontally (i.e. flat or parallel, or substantially parallel, to the skin surface, e.g. the brain's cortex) inside the housing. Hence, the sum of the postsynaptic potentials of the different pyramidal neurons oriented vertically to the brain cortex will be captured homogeneously by means of the whole surface of the electrode plate.

The first hole has a conical shape for injecting and replacing an electroconductive gel inside the hollow space. The first protruding part is disposed in the housing inclined outwardly at an angle between 50-degree and 80-degree with respect to the contact surface of the electrode device (i.e. the bottom surface of the electrode device contacting the skin surface of the patient).

The proposed electrode device has a size and form adaptable to the head of a newborn. It is connected with a short wire that, once disconnected from an amplifier, facilitates the manipulation of the infant by the healthcare staff. The vertical orientation of the insertion between the electrode plate and the wire improves stability in the lying position. The conical hole prevents accidental contact of a blunt tip of a needle with the infant's scalp and allows the safe replacement of the electroconductive gel, thus facilitating the good signal quality during long-term recordings. Furthermore, by being a surface electrode, it does not cause discomfort to the patient.

In an embodiment, the electrode plate comprises a first gap which allows the electroconductive gel to flow through and a second gap to fix the rotating position of the electrode plate to the housing using a clamping element. Hence the distance between the skin surface and the electrode plate is reduced (i.e. the mentioned hollow space is smaller).

In a particular embodiment, the first protruding part is disposed at a 60-degree angle with respect to the contact surface of the electrode device.

The first protruding part can include a plug for closure of the first hole. Hence, the properties of the electroconductive gel can be kept for a longer period of time.

The housing can be made of a polymer, ceramic or metallic material. In a particular embodiment, the housing is made of a thermoplastic Polyether-Polyurethane elastomer. The electrode plate can be made of, or can comprise a coating made of, Silver (Ag), Gold (Au), Silver/Silver-Chloride (Ag/AgCI), or any good electrical conductor material (e.g. Tin, Stainless Steel, Platinum, Cooper, Lead, or Nichrome). In other embodiments, the electrode plate can be made of, or can comprise a coating made of, a conductive polymer such Liquid Cristal Polymer (LCP), Polyether ether ketone (PEEK), Polyphenylsulfone (PPSU, PPSF), ultra-high molecular weight polyethylene, among others; therefore the electrode is fully MRI-compatible. In any case, for the electrode being fully MRI-compatible any conductive and non-magnetic material can be used.

In yet other embodiments, the electrode besides being fully MRI-compatible can be also radiolucent. In this particular case the wire is preferably made of one or more carbon fibers.

In an embodiment, the adhesive element is a flexible adhesive pad including an adhesive layer provided on a lower or bottom surface of the adhesive element. Alternatively or complementarily, the adhesive element comprises a double-sided tape.

In an embodiment, the electrode device also has a connector such as a touchproof connector or a male snap connector, among others, arranged on a distal end of the wire connector.

In an embodiment, the length of the wire connection is less than 50 millimeters, particularly of approximately 30 millimeters. In other embodiments, the length of the wire connection is between 50 millimeters and 600 millimeters, preferably 100 millimeters. In yet other embodiments, the length of the wire connection can be between 0.6 and 2 meters.

In some embodiments, the wire connection can have a spiral/curved shape in order to have an elastic behavior that will absorb some axial force from the wires of a bioelectric signal amplifier.

In some embodiments, the wire connection can also include an insulating layer, for example made of a material comprising silicone, Polyvinyl chloride, Polypropylene, Polyethylene, XLPE (Cross-Linked Polyethylene), EPR (Ethylene Propylene Rubber), ECTFE (ethylene chlorotrifluoroethylene), PVDF (Polyvinylidene fluoride), Nylon, CPE (Chlorinated polyethylene), etc.

In yet some embodiments, one or more sealing elements can be disposed between the housing and the insulating layer of the wire connection and/or over the housing and the first protruding part/first hole.

In an embodiment, the surface of the housing in contact with the adhesive element has a curvature that better fits with the skull shape.

In some embodiments, the electrode plate support surface is formed by several supports that sustain the electrode plate horizontally disposed (i.e. parallel or substantially parallel to the skin surface of the patient). In addition, the electrode device also includes an encapsulating/isolation element positioned on top of the electrode plate. The encapsulating element prevents the gel from contacting the wire connection, thus avoiding galvanic corrosion.

The electrode device comprises a third protruding part that is symmetrically arranged with regard to the first protruding part. The third protruding part has also a conical-shaped hole particularly for replacing the electroconductive gel present in the hollow space.

Hence, the proposed electrode device allows an easy electrode placement as well as a wire cable extension that permits an easier electrical connection. The electrode device design also allows the patient, in particular newborns, to rotate the head without difficulties. The new design allows replacing the electroconductive gel for long-term aEEG recordings.

### Brief Description of the Drawings

The previous and other advantages and features will be more fully understood from the following detailed description with reference to the attached figures, which must be considered in an illustrative and non-limiting manner, in which:
Figs. 1A-1C illustrate different views of an electrode device.
Figs. 2A-2B illustrate different views of another electrode device.
Fig. 3 illustrates another electrode device. In this case, the surface of the housing configured to contact the adhesive element is curved. The adhesive element comprises a double-sided tape.
Figs. 4A-4B illustrate another device in which the surface of the housing configured to contact the adhesive element is curved. In this case the adhesive element comprises a double-sided tape. Fig. 4B is an enlarged view of section B outlined in Fig. 4A.
Figs. 5A-5E illustrate different views of another electrode device.
Figs. 6A and 6B illustrate different views of an embodiment of the proposed electrode device according to the invention.

### Detailed Description

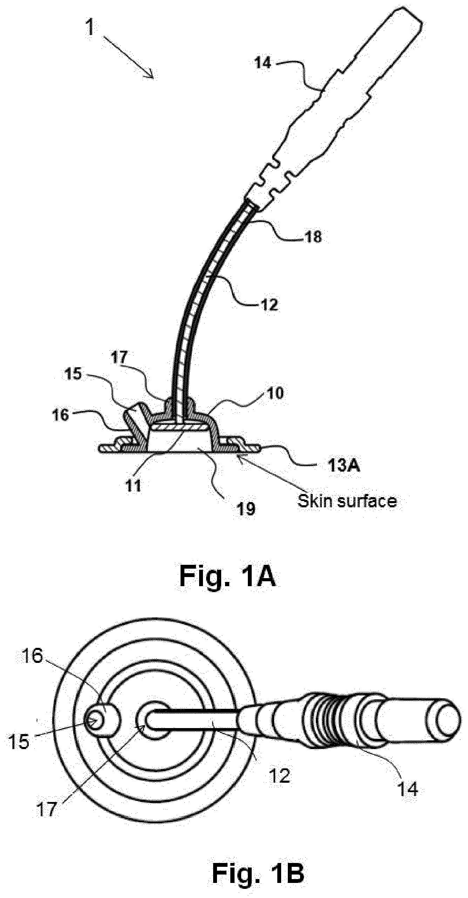

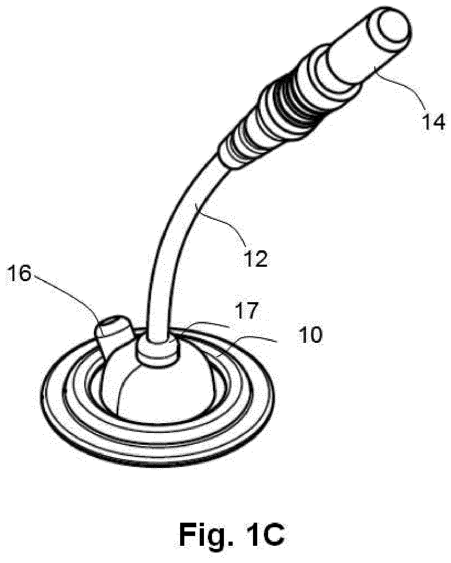

With reference to Figs. 1A-1C, therein a first electrode device is showed. The electrode device 1 of this embodiment includes a housing 10, for example made of a polymeric, ceramic or metallic material, which is mounted or arranged on a flexible adhesive pad 13A to adhere the electrode device 1 to a skin surface of a patient. The flexible adhesive pad 13A has an adhesive layer provided on a lower/bottom surface thereof to allow said adhesion of the electrode device 1 to the skin surface. As seen in the figures, the flexible adhesive pad 13A has a larger diameter than the housing 10.

The electrode device 1 also includes an electrode plate 11 horizontally disposed (i.e. parallel or substantially parallel to the skin surface of the patient) inside the housing 10 at a certain distance therein forming a hollow space 19. In particular, the electrode plate 11 is made of, or comprises a coating made of, Silver (Ag), Gold (Au), or Silver/Silver-Chloride (Ag/AgCI). In other embodiments however any other good electrical conductor material such as Tin, Stainless Steel, Platinum, Cooper, Lead, or Nichrome, among others, can be used. In some other embodiments, the electrode device 1 is fully MRI-compatible and/or radiolucent. In this case, conductive and non-magnetic materials should be used for the electrode plate 11 and/or wire connection 12.

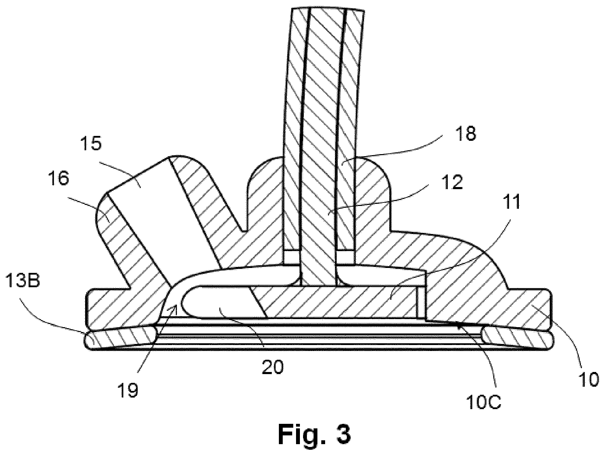

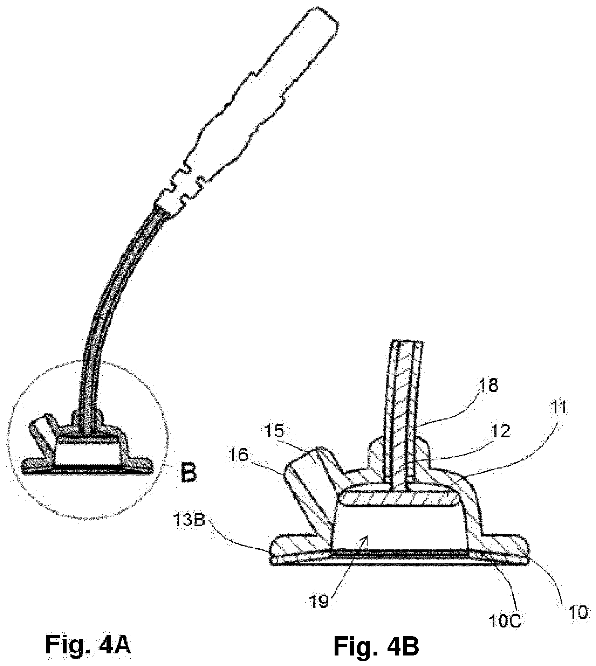

Also, the electrode device 1 includes a flexible wire connection 12 for the electrode plate 11. The wire connection 12 is fixed to the electrode plate 11 by one of its extremes using at least one welding point, the other extreme being attached to a touchproof connector 14. It should be noted that in other embodiments, in this case not illustrated, the connector used can be of different type, for example a male snap connector, among others. Likewise, it should be noted that in other embodiments, an adhesive element comprising a double-sided tape 13B (see Fig. 3 and Figs. 4A and 4B for example) can be used instead of the flexible adhesive pad 13A. In yet other embodiments, the adhesive element comprises a hydrocolloid adhesive; thus the electrode device 1 is for single-patient use and it is disposable.

In some embodiments, the housing 10 is translucent and flexible. By being translucent skin check (irritation), gel check (from drying or spilling) and electrode plate check (from movements or change in color for checking the Ag/AgCI coating, corrosion...) are easily possible. By being flexible a better interaction with the skin is further enabled (skin injures due to the reduction to stress concentrations caused by rigid materials is avoided).

The housing 10 has two protruding parts 16, 17. In this particular embodiment, the first protruding part 16 is located inclined outwardly at an angle of approximately 60º-65º with regard to the longitudinal direction of the skin surface (or with regard to the contact surface of the electrode device 1). It should be noted that in other embodiments, in this case not illustrated, the first protruding part 16 can be located at an angle between 50º and 80º with respect to the contact surface of the electrode device 1. The first protruding part 16 includes a conical-shaped hole 15 to allow the injection and removal of an electroconductive gel or hydrogel. The second protruding part 17 is located about the axis of the housing 10 and includes a second hole for the upright introduction of the wire connection 12.

Any biocompatible electroconductive gel can be used in accordance with the present disclosure. The electroconductive gel can be replaced via a blunt needle through the conical-shaped hole 15 but the needle is blocked by the geometry of the hole to avoid skin injuries.

Particularly, the electrode device 1 of Figs. 1A-1C also includes an insulting layer 18 surrounding the wire connection. Different materials can be used as insulating layer for example silicone, Polyvinyl chloride, Polyethylene, Polyvinylidene fluoride and/or Polypropylene, among others.

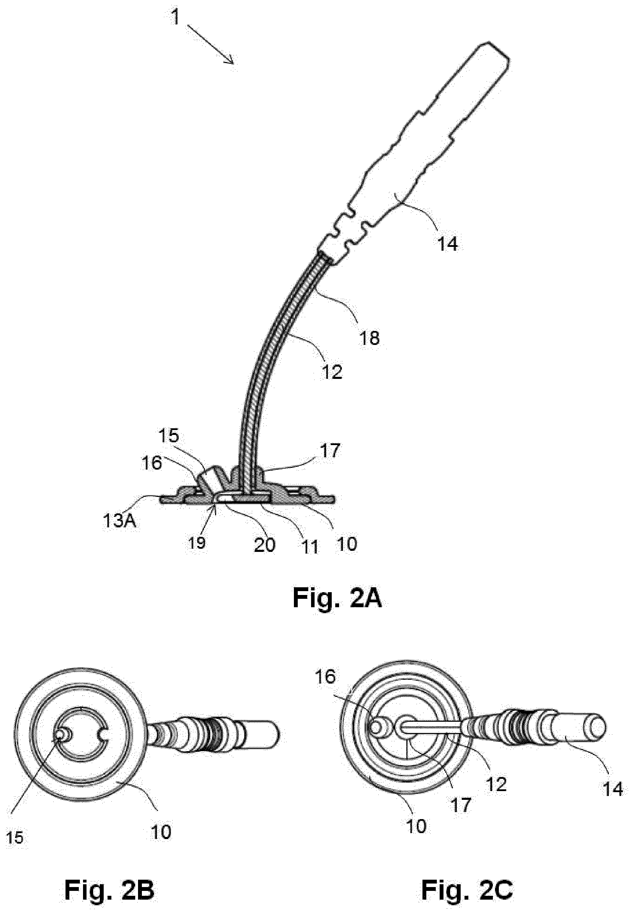

With regard to Figs. 2A-2C, therein another electrode device 1 is illustrated. In this case, different to the embodiment of Figs. 1A-1C, the hollow space 19 is reduced since the electrode plate 11 is pierced in two parts: one opening 20 is disposed to fit with the conical-shaped hole 15 to allow the electroconductive gel to flow through; the other opening (not seen in the figures) is used for the fixation of the electrode plate 11 to the housing 10, for example via a clamping or fixing element.

Fig. 3 illustrates an example in which a bottom surface 10C of the housing 10 that contacts the adhesive element comprises a curvature to better fit with the skull. The size of the housing 10 in this case is reduced. The adhesive element comprises a double-sided tape 13B.

Figs. 4A and 4B illustrate another example in which the bottom surface 10C of the housing 10 is curved. In this embodiment, the adhesive element comprises a double-sided tape 13B and the electrode plate 11 is not pierced.

In some examples, sealing elements can be also disposed between the housing 10 and the insulating layer 18 of the wire connection 12. In some embodiments also, the first protruding part 16 can comprise a plug for closure thereof.

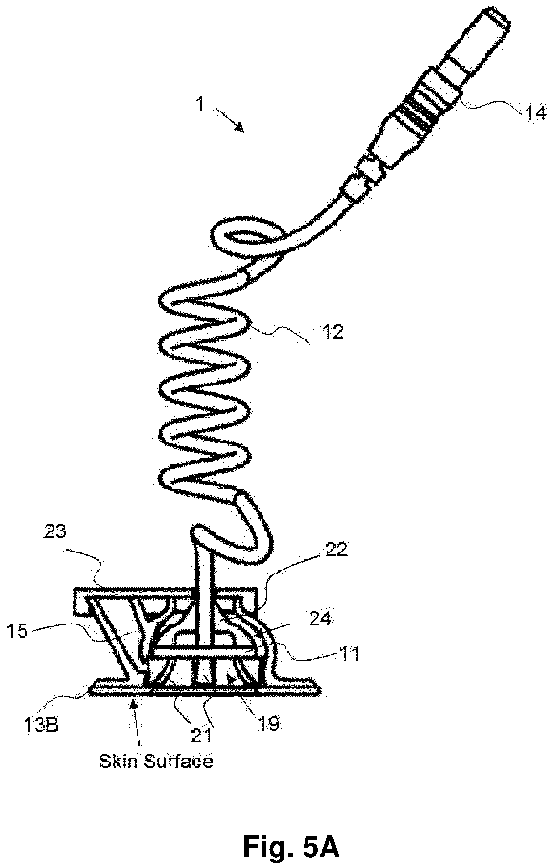

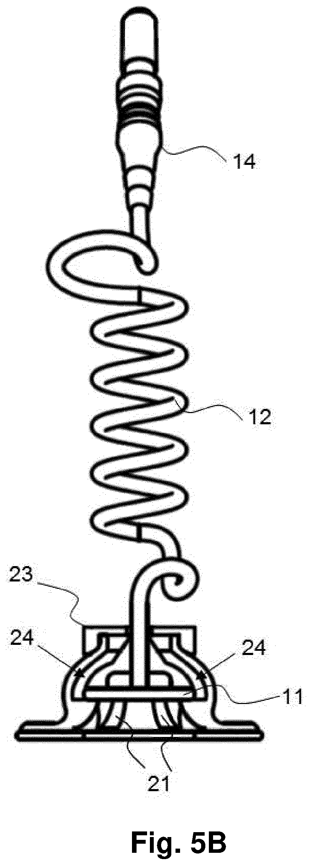

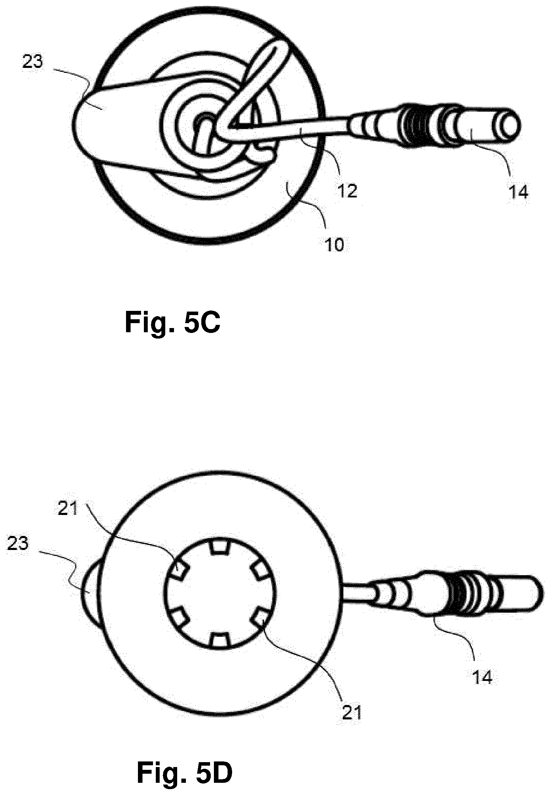

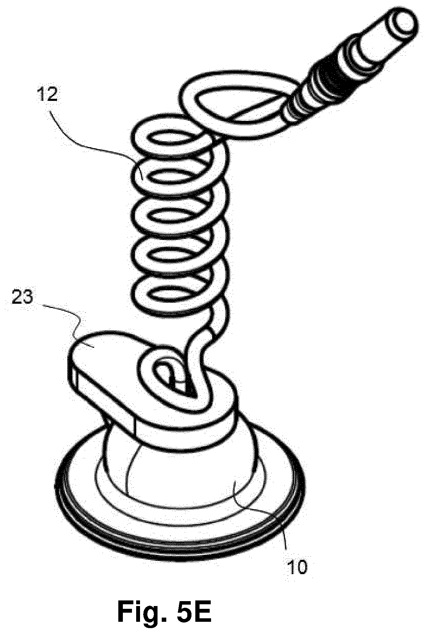

With regard to Figs. 5A-5E, therein another electrode device 1 is shown. According to this embodiment, the wire connection 12 has a curved/spiral shape and the support surface comprises a plurality of supporting structures (or simply supports) 21 that hold the electrode plate 11. The electrode device 1 (see the two different sections of Figs. 5A and 5B) also has an encapsulating element 22 positioned on top of the electrode plate 11 that isolates the wire-plate connection for the electroconductive gel. A gap 24 between the encapsulating element 22 and the housing 10 allows the electroconductive gel to flow from the hollow space 19 between the electrode plate 11 and the skin to the second protruding part 17. A sealing element 23 is also included covering an upper part of the housing 10 and the first hole 15, such that drying of the gel is reduced. To replace the electroconductive gel, the sealing element 23 has to be lifted to release the first protruding part 16 to insert a blunt needle and to visually check the drain of the gel through the second protruding part 17.

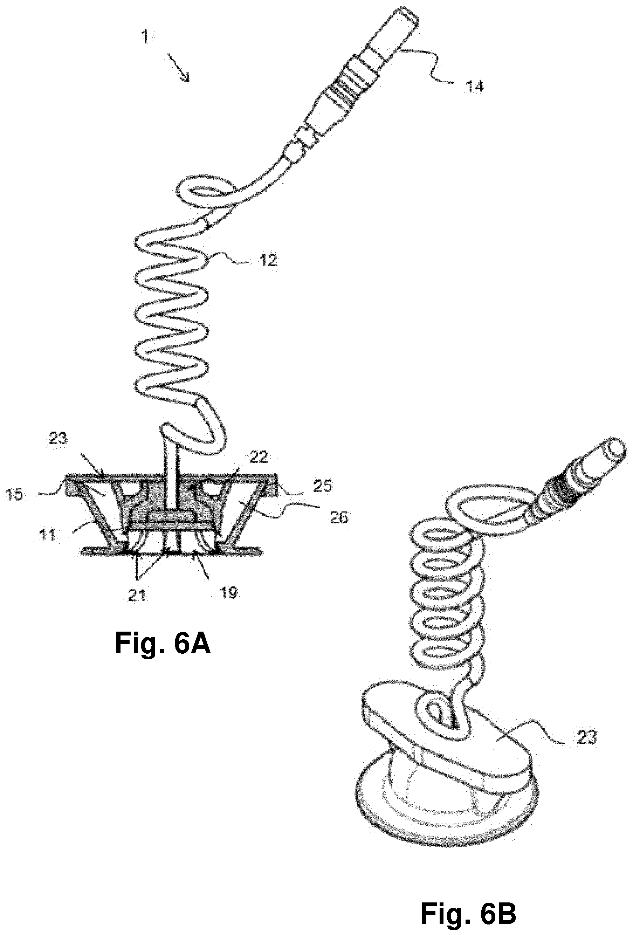

Figs. 6A and 6B illustrate an embodiment of the proposed electrode device 1 according to the invention.

The electrode device 1 of this embodiment is similar to the electrode device 1 of previous figures 5A-5C but in this case the housing 10 besides the two protruding parts 16, 17 described above further has a (third) protruding part 25, which is symmetric to the protruding part 16. The protruding part 25 also includes a conical-shaped hole 26. The third protruding part 25 allows an easier replacement of the electroconductive gel in case the viscosity of the gel is too low, the gel used dries fast or in case the gel only remains in the bottom surface of the electrode plate 11. In this embodiment, to replace the electroconductive gel, after lifting the sealing element 23, a blunt needle can be inserted in the first 16 or third 25 protruding part and drained by the other one.

It will be understood that various details of the presently disclosed matter may be changed.

Furthermore, the previous description is for the purpose of illustration only, and not for the purpose of limitation.

As used herein, the term "about" and/or "substantially", when referring to a value or to feature, are meant to encompass variations of in some examples ±10%, in some examples ±5%, from the specified value or feature, as such variations are appropriate to perform the disclosed device.

The scope of the present invention is defined in the following set of claims.

## Claims

1. An electrode device for amplitude-integrated electroencephalography (aEEG), comprising:
a housing (10) mounted on an adhesive element (13A, 13B) to adhere the electrode device (1) to a skin surface of a patient;
an electrode plate (11) placed in the housing (10) at a certain distance from a support surface of the housing (10) forming a hollow space (19);
a wire connection (12) for the electrode plate (11), said wire connection (12) having a given length and comprising a proximal end and a distal end;
the housing (10) comprising a first protruding part (16) and a second protruding part (17), the first protruding part (16) including a first hole (15), and the second protruding part (17) including a second hole through which the proximal end of the wire connection (12) is introduced and attached to the electrode plate (11), the second protruding part (17) being disposed on the axis of the housing (10),
wherein:
the device further comprises a third protruding part (25) that is symmetrically arranged with regard to the first protruding part (16); and
the first protruding part (16) is disposed in the housing (10) inclined outwardly at an angle between 50-degree and 80-degree with respect to a contact surface of the electrode device, the first hole (15) having a conical-shaped geometry and being configured to inject an electroconductive gel inside the hollow space (19), and the third protruding part (25) including a conical-shaped hole (26) configured to remove the electroconductive gel from the hollow space (19).

2. The electrode device of claim 1, wherein the first protruding part (16) is disposed at a 60 or 65 degree angle with respect to the contact surface of the electrode device.

3. The electrode device of any one of the previous claims, wherein the wire connection (12) further comprises a connector (14) on said distal end.

4. The electrode device of any one of the previous claims, wherein the adhesive element is a flexible adhesive pad (13A) comprising an adhesive layer provided on a lower surface thereof.

5. The electrode device of any one of the previous claims 1 to 3, wherein the adhesive element is a double-sided tape (13B).

6. The electrode device of any one of the previous claims, wherein the attachment of the wire connection (12) to the electrode plate (11) comprises one or more welding points.

7. The electrode device of any one of the previous claims, wherein the wire connection (12) further comprises an insulating layer (18).

8. The electrode device of any one of the previous claims 1 to 7, wherein the length of the wire connection (12) is between 0.05 meters and 2 meters.

9. The electrode device of any one of the previous claims, wherein:
the housing (10) is made of a polymer, ceramic or metallic material; and
the electrode plate (11) is made of, or comprises a coating made of, Silver (Ag), Gold (Au), Silver/Silver-Chloride (Ag/AgCI), Tin, Stainless Steel, Platinum, Cooper, Lead, Nichrome, or a conductive non-magnetic polymer.

10. The electrode device of any one of the previous claims 1 to 7, wherein the first protruding part (16) further comprises a plug for closure thereof.

11. The electrode device of any one of the previous claims, wherein the electrode plate (11) comprises a first gap adapted to allow the electroconductive gel to flow through and a second gap adapted to fix a rotating position of the electrode plate (11) to the housing (10) using a clamping element.

12. The electrode device of claim 1, wherein the housing (10) is made of a translucent and flexible material.

13. The electrode device of any one of the previous claims, wherein the wire connection (12) has a spiral shape.

14. The electrode device of any one of the previous claims, wherein the support surface comprises a plurality of supporting structures (21) configured to sustain the electrode plate (11), and the electrode device (1) further comprises an encapsulating element (22) positioned on top of the electrode plate (11).

## Patentansprüche

1. Elektrodenvorrichtung für eine amplitudenintegrierte Elektroenzephalographie (aEEG), die umfasst:
ein Gehäuse (10), das an einem Haftelement (13A, 13B) angebracht ist, um die Elektrodenvorrichtung (1) an einer Hautoberfläche eines Patienten anzuhaften;
eine Elektrodenplatte (11), die in dem Gehäuse (10) in einem gewissen Abstand zu einer Trägerfläche des Gehäuses (10) platziert ist, wobei ein Hohlraum (19) gebildet wird;
eine Drahtverbindung (12) für die Elektrodenplatte (11), wobei die Drahtverbindung (12) eine bestimmte Länge aufweist und ein proximales Ende und ein distales Ende umfasst;
das Gehäuse (10) einen ersten vorstehenden Teil (16) und einen zweiten vorstehenden Teil (17) umfasst, wobei der erste vorstehende Teil (16) ein erstes Loch (15) aufweist und der zweite vorstehende Teil (17) ein zweites Loch aufweist, durch das das proximale Ende der Drahtverbindung (12) eingeführt und an der Elektrodenplatte (11) befestigt wird, wobei der zweite vorstehende Teil (17) auf der Achse des Gehäuses (10) angeordnet ist,
wobei:
die Vorrichtung ferner einen dritten vorstehenden Teil (25) umfasst, der in Bezug auf den ersten vorstehenden Teil (16) symmetrisch angeordnet ist; und
der erste vorstehende Teil (16) in dem Gehäuse (10) in einem Winkel zwischen 50 Grad und 80 Grad in Bezug auf eine Kontaktfläche der Elektrodenvorrichtung nach außen geneigt angeordnet ist, wobei das erste Loch (15) eine konisch geformte Geometrie aufweist und dazu ausgestaltet ist, ein elektrisch leitendes Gel in den Hohlraum (19) zu spritzen, und wobei der dritte vorstehende Teil (25) ein konisch geformtes Loch (26) aufweist, das dazu ausgestaltet ist, das elektrisch leitende Gel aus dem Hohlraum (19) zu entfernen.

2. Elektrodenvorrichtung nach Anspruch 1, wobei der erste vorstehende Teil (16) in einem Winkel von 60 oder 65 Grad in Bezug auf die Kontaktfläche der Elektrodenvorrichtung angeordnet ist.

3. Elektrodenvorrichtung nach einem der vorstehenden Ansprüche, wobei die Drahtverbindung (12) ferner ein Verbindungselement (14) an dem distalen Ende umfasst.

4. Elektrodenvorrichtung nach einem der vorstehenden Ansprüche, wobei das Haftelement ein flexibles Haftpad (13A) ist, das eine auf einer Unterseite davon bereitgestellte Haftschicht umfasst.

5. Elektrodenvorrichtung nach einem der vorstehenden Ansprüche 1 bis 3, wobei das Haftelement ein doppelseitiges Klebeband (13B) ist.

6. Elektrodenvorrichtung nach einem der vorstehenden Ansprüche, wobei die Befestigung der Drahtverbindung (12) an der Elektrodenplatte (11) einen oder mehrere Schweißpunkte umfasst.

7. Elektrodenvorrichtung nach einem der vorstehenden Ansprüche, wobei die Drahtverbindung (12) ferner eine Isolierschicht (18) umfasst.

8. Elektrodenvorrichtung nach einem der vorstehenden Ansprüche 1 bis 7, wobei die Länge der Drahtverbindung (12) zwischen 0,05 Metern und 2 Metern beträgt.

9. Elektrodenvorrichtung nach einem der vorstehenden Ansprüche, wobei
das Gehäuse (10) aus einem Polymer-, Keramik- oder metallischen Material hergestellt ist; und
die Elektrodenplatte (11) aus einer Beschichtung aus Silber (Ag), Gold (Au), Silber/Silberchlorid (Ag/AgCl), Zinn, Edelstahl, Platin, Kupfer, Blei, Nickelchrom oder einem leitenden nichtmagnetischen Polymer besteht oder dies umfasst.

10. Elektrodenvorrichtung nach einem der vorstehenden Ansprüche 1 bis 7, wobei der erste vorstehende Teil (16) ferner einen Stopfen zum Verschließen davon umfasst.

11. Elektrodenvorrichtung nach einem der vorstehenden Ansprüche, wobei die Elektrodenplatte (11) einen ersten Spalt umfasst, der dazu ausgelegt ist, das Hindurchfließen des elektrisch leitenden Gels zu ermöglichen, und einen zweiten Spalt umfasst, der dazu ausgelegt ist, eine Drehposition der Elektrodenplatte (11) in Bezug auf das Gehäuse (10) unter Verwendung eines Klemmelements zu fixieren.

12. Elektrodenvorrichtung nach Anspruch 1, wobei das Gehäuse (10) aus einem transluzenten und flexiblen Material hergestellt ist.

13. Elektrodenvorrichtung nach einem der vorstehenden Ansprüche, wobei die Drahtverbindung spiralförmig ist.

14. Elektrodenvorrichtung nach einem der vorstehenden Ansprüche, wobei die Trägerfläche eine Mehrzahl von Trägerstrukturen (21) umfasst, die dazu ausgestaltet sind, die Elektrodenplatte (11) zu stützen, und wobei die Elektrodenvorrichtung (1) ferner ein Einbettungselement (22) umfasst, das oben auf der Elektrodenplatte (11) positioniert ist.

## Revendications

1. Dispositif d'électrode pour électroencéphalographie intégrée en amplitude (aEEG), comprenant :
un boîtier (10) monté sur un élément adhésif (13A, 13B) pour faire adhérer le dispositif d'électrode (1) à une surface cutanée d'un patient ;
une plaque d'électrode (11) placée dans le boîtier (10) à une certaine distance d'une surface de support du boîtier (10) formant un espace creux (19) ;
une connexion de fil (12) pour la plaque d'électrode (11), ladite connexion de fil (12) ayant une longueur donnée et comprenant une extrémité proximale et une extrémité distale ;
le boîtier (10) comprenant une première partie en saillie (16) et une deuxième partie en saillie (17), la première partie en saillie (16) comprenant un premier trou (15), et la deuxième partie en saillie (17) comprenant un deuxième trou à travers lequel l'extrémité proximale de la connexion de fil (12) est introduite et fixée à la plaque d'électrode (11), la deuxième partie en saillie (17) étant disposée sur l'axe du boîtier (10),
dans lequel :
le dispositif comprend en outre une troisième partie en saillie (25) qui est disposée symétriquement par rapport à la première partie en saillie (16) ; et
la première partie en saillie (16) est disposée dans le boîtier (10) inclinée vers l'extérieur à un angle compris entre 50 degrés et 80 degrés par rapport à une surface de contact du dispositif d'électrode, le premier trou (15) ayant une géométrie de forme conique et étant configuré pour injecter un gel électroconducteur à l'intérieur de l'espace creux (19), et la troisième partie en saillie (25) comprenant un trou de forme conique (26) configuré pour retirer le gel électroconducteur de l'espace creux (19).

2. Dispositif d'électrode selon la revendication 1, dans lequel la première partie en saillie (16) est disposée à un angle de 60 ou 65 degrés par rapport à la surface de contact du dispositif d'électrode.

3. Dispositif d'électrode selon l'une quelconque des revendications précédentes, dans lequel la connexion de fil (12) comprend en outre un connecteur (14) sur ladite extrémité distale.

4. Dispositif d'électrode selon l'une quelconque des revendications précédentes, dans lequel l'élément adhésif est un tampon adhésif flexible (13A) comprenant une couche adhésive disposée sur une surface inférieure de celui-ci.

5. Dispositif d'électrode selon l'une quelconque des revendications précédentes 1 à 3, dans lequel l'élément adhésif est un ruban adhésif double face (13B).

6. Dispositif d'électrode selon l'une quelconque des revendications précédentes, dans lequel la fixation de la connexion de fil (12) à la plaque d'électrode (11) comprend un ou plusieurs points de soudure.

7. Dispositif d'électrode selon l'une quelconque des revendications précédentes, dans lequel la connexion de fil (12) comprend en outre une couche isolante (18).

8. Dispositif d'électrode selon l'une quelconque des revendications précédentes 1 à 7, dans lequel la longueur de la connexion de fil (12) est comprise entre 0,05 mètre et 2 mètres.

9. Dispositif d'électrode selon l'une quelconque des revendications précédentes, dans lequel :
le boîtier (10) est constitué d'un matériau polymère, céramique ou métallique ; et
la plaque d'électrode (11) est constituée par ou comprend un revêtement constitué par, l'argent (Ag), l'or (Au), l'argent/le chlorure d'argent (Ag/AgCl), l'étain, l'acier inoxydable, le platine, le cuivre, le plomb, le nichrome ou \un polymère conducteur non magnétique.

10. Dispositif d'électrode selon l'une quelconque des revendications précédentes 1 à 7, dans lequel la première partie en saillie (16) comprend en outre un bouchon pour sa fermeture.

11. Dispositif d'électrode selon l'une quelconque des revendications précédentes, dans lequel la plaque d'électrode (11) comprend un premier espace adapté pour permettre au gel électroconducteur de s'écouler et un deuxième espace adapté pour fixer une position de rotation de la plaque d'électrode (11) au boîtier (10) à l'aide d'un élément de serrage.

12. Dispositif d'électrode selon la revendication 1, dans lequel le boîtier (10) est constitué d'un matériau translucide et flexible.

13. Dispositif d'électrode selon l'une quelconque des revendications précédentes, dans lequel la connexion de fil (12) a une forme en spirale.

14. Dispositif d'électrode selon l'une quelconque des revendications précédentes, dans lequel la surface de support comprend une pluralité de structures de support (21) configurées pour soutenir la plaque d'électrode (11), et le dispositif d'électrode (1) comprend en outre un élément d'encapsulation (22) positionné au-dessus de la plaque d'électrode (11).
